(19) Europäisches Patentamt
European Patent Office
Office européen des brevets

(11) Publication number: **0 470 668 A1**

# (12) EUROPEAN PATENT APPLICATION

(21) Application number: **91202005.4**

(22) Date of filing: **03.08.91**

(51) Int. Cl.[5]: **C07D 453/02**

(30) Priority: **08.08.90 GB 9017353**

(43) Date of publication of application:
**12.02.92 Bulletin 92/07**

(84) Designated Contracting States:
**CH DE FR GB IT LI NL**

(71) Applicant: **MERCK SHARP & DOHME LTD.**
**Hertford Road**
**Hoddesdon Hertfordshire EN11 9BU(GB)**

(72) Inventor: **Houghton, Peter**
**8 Tower Close, Bassingbourn**
**Nr.Royston, Hertfordshire(GB)**
Inventor: **Humphrey, Guy R.**
**14 Walker Drive**
**Bell Mead, NJ 08502(US)**

(74) Representative: **Barrett-Major, Julie Diane et al**
**Merck & Co., Inc. European Patent Department Terlings Park Eastwick Road**
**Harlow Essex CM20 2OR(GB)**

## (54) A process for preparing enantiomers of compounds having muscarinic activity.

(57) A process for preparing a substantially pure enantiomer of a compound of formula (I) or a salt or prodrug thereof:

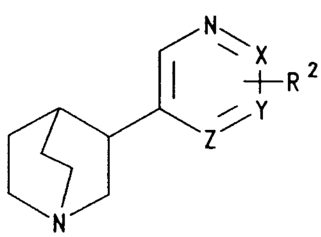

( I )

wherein one of X, Y and Z is an N atom and the remainder are C atoms; and
$R^2$ is hydrogen, halo, -CN, $-COOR^6$, $-CONR^6R^7$, or a saturated or unsaturated, substituted or unsubstituted hydrocarbon group, wherein $R^6$ and $R^7$ are independently selected from hydrogen and $C_{1-2}$ alkyl;
which process comprises cyclisation of an enantiomer of a compound of formula (19A) or salt thereof:

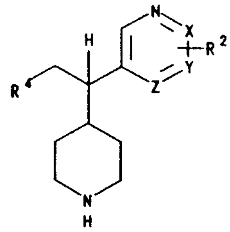

(19A)

EP 0 470 668 A1

wherein $R^2$, X, Y and Z are as defined in formula (I); and $R^4$ is a labile leaving group is disclosed. Optionally, the compound so prepared may be converted to a salt or prodrug thereof. A preferred compound is (R)-(-)-3-(6-chloropyrazin-2-yl)-1-azabicyclo[2.2.2]octane or a salt thereof.

The present invention relates to a process for preparing enantiomers of compounds having muscarinic agonist activity.

In European published patent specification no. 327155 are disclosed certain azabicyclic compounds, including pyrazine derivatives thereof, having, for example, muscarinic agonist activity, and processes for their preparation. However, no specific enantiomers of the compounds are disclosed and no process is disclosed wherein the optical isomers of the final azabicyclic compounds can separately be prepared.

It was then surprising to find a new stereo-selective process, starting with a commercially - available compound which could be used to prepare the desired final enantiomers without undertaking a resolution in the final step, and having other advantages including that of a higher yield resulting from this process compared with a process requiring resolution of the final racemates.

Thus, the present invention provides a process for preparing a substantially pure enantiomer of a compound of formula (I)

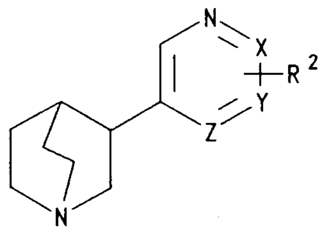

(I)

one of X, Y and Z is an N atom and the remainder are C atoms; and

$R^2$ is hydrogen, halo, -CN, $-COOR^6$, $-CONR^6R^7$, or a saturated or unsaturated, substituted or unsubstituted hydrocarbon group, wherein $R^6$ and $R^7$ are independently selected from hydrogen and $C_{1-2}$ alkyl;

which process comprises cyclisation of an enantiomer of a compound of formula (19A) or salt thereof:

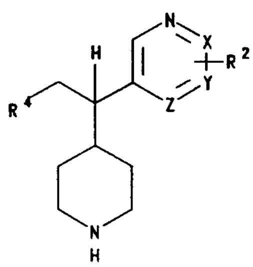

(19A)

wherein

$R^2$, X, Y and Z are as defined in formula (I);

and

$R^4$ is a labile leaving group such as mesylate ($OS(O)_2CH_3$), lower alkanoate such as acetate or halo such as chloro or bromo.

(Step a) Cyclisation of (19A) is carried out in a pH range of from 8 to 10, such as in a two-phase system comprising a mild inorganic base such as an aqueous alkali or alkaline earth metal carbonate, hydrogen carbonate or hydroxide such as sodium or potassium carbonate or hydrogencarbonate or barium hydroxide; preferably aqueous potassium carbonate. The non-aqueous phase is any in which the compound of formula (I) so prepared is soluble, for example, solvents having a b.p. $\geq 60^\circ C$ such as a lower alkanol or ester such as t-butanol or ethyl acetate, or an ether such as diisopropyl ether, or toluene. For a single-phase system, any alcohol and water can be used, for example t-butanol/water.

The following scheme shows how the compound of formula (19A) can be prepared in accordance with this invention.

The compound of formula (19A) is usually in a salt form such as the hydrochloride salt as it is prepared by deprotecting the corresponding enantiomer of a compound of formula (19):

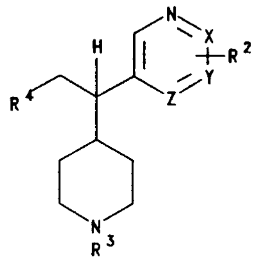

(19)

wherein $R^2$, X, Y, Z and $R^4$ are as defined in formula (19A); and

$R^3$ is a secondary amine blocking group which is removable under mild conditions. Suitable blocking or protecting groups are known to those skilled in the art and include t-butoxycarbonyl or benzyloxycarbonyl.

(Step b) Removal of the blocking group $R^3$ to form unprotected compound (19A) is carried out by conventional techniques known to those skilled in the art, for example, by reaction with trifluoroacetic acid or a hydrogen halide such as hydrogen bromide or hydrogen chloride. For this purpose, the reaction is preferably carried out in solution in a suitable solvent such as t-butanol, or a lower alkyl organic acid or ester such as acetic acid, or ethyl or iso-propyl acetate. Preferably, crude compound (19) is dissolved in ethyl acetate and dry HCl gas bubbled through.

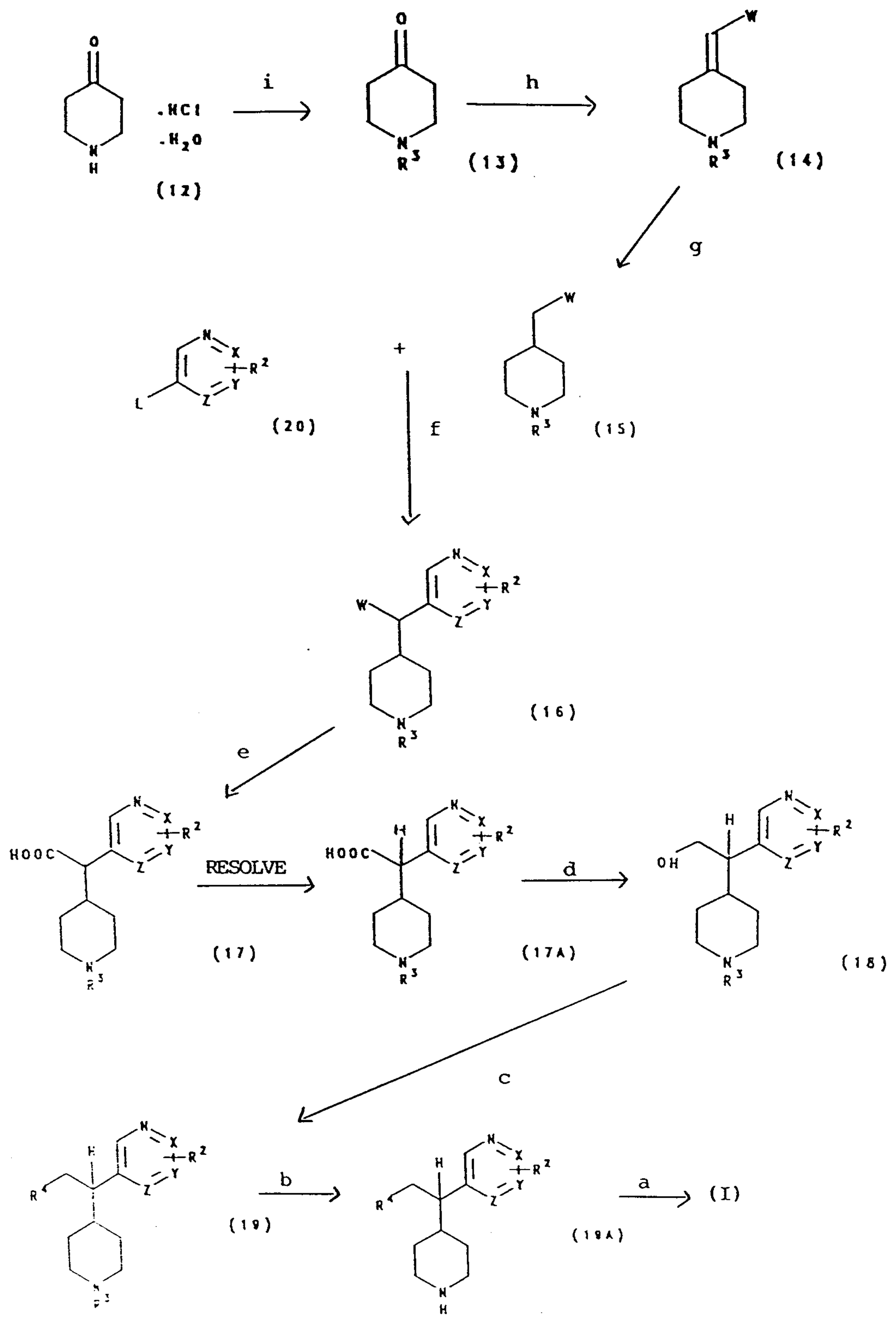


SCHEME

The enantiomer of the compound of formula (19) may be prepared by conventional techniques from the corresponding hydroxy analogue of an enantiomer formula (18):

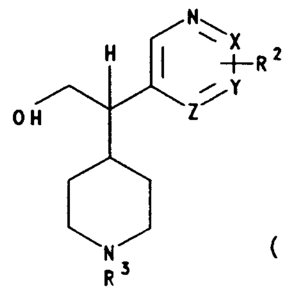

(18)

wherein $R^2$, X, Y, Z and $R^3$ are as defined in formula (19).

(Step c) Conversion of the hydroxy group in the alcohol (18) for a suitable leaving group $R^4$ to form (19) may be undergone by methods analogous to those known in the art. For example, mesylation ($R^4 = OS(O)_2CH_3$) can take place by addition of methane sulphonyl chloride to the alcohol (18) in a suitable inert organic solvent such as ethyl acetate under cooling in the presence of a lower alkyl amine such as triethylamine. Preferably, the reaction is carried out under a nitrogen atmosphere at from 0° to -20° C.

When $R^4$ is halo, bromination may be undertaken by phosphorous tribromide or chlorination by thionyl chloride in a solvent such as an ether or tetrahydrofuran. Optionally, the mesylate may be formed first and then converted to halo. Indeed, any leaving group may be converted to any other suitable leaving group by conventional methods.

The enantiomer of the alcohol (18) is itself prepared by reduction of the corresponding chiral acid of formula (17A) or a salt thereof:

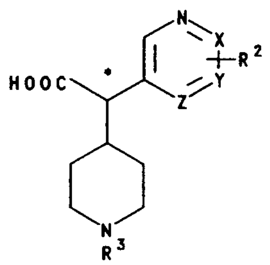

(17A)

wherein $R^2$, X, Y, Z and $R^3$ are as defined in formula (18); and the salt is preferably a suitable organic amine salt such as α-methylbenzylamine.

(Step d) The chiral acid (17A) is reduced to the alcohol (18) by a conventional reducing agent such as borane, preferably in the presence of an inert organic solvent such as tetrahydrofuran or an ether under an inert atmosphere such as nitrogen, preferably at a reduced temperature of about -20° - +5° C.

The chiral acid of formula (17A) is prepared by resolution of the corresponding racemate of formula (17)

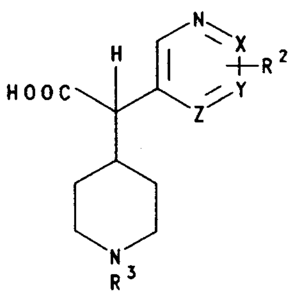

(17)

(wherein $R^2$, X, Y, Z and $R^3$ are as defined in formula (17A)) by classical resolution techniques known to those skilled in the art. For example, to obtain the (+)-acid of formula (17A), an organic amine such as (L)-(-)-α-methyl-benzylamine may be used. The resolution is undertaken in any inert organic solvent such as ethers or acetates such as isopropyl acetate or ethyl acetate.

Racemisation to maximise the yield of the desired chiral acid may also be carried out by methods

analogous to those known in the art. A preferred base is triethylamine, but others such as DBU or DABCO may also be used. Suitable solvents include those mentioned above for the resolution step and the racemisation is preferably carried out at the boiling temperature of the solvent, i.e. in the range of from $50^{\circ}$ - $100^{\circ}$ C, most preferably at around $80^{\circ}$ C. More preferably, the acid (17/17A) may be in protected form, for example, with trimethyl-silylchloride or the like, to inhibit unwanted by-products.

The acid (17) is prepared by converting the corresponding compound of formula (16):

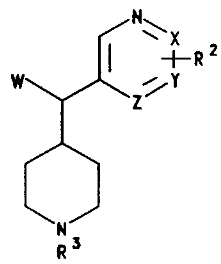

(16)

wherein $R^2$, X, Y, Z and $R^3$ are as defined in formula (17); and

W is cyano or a reactive derivative of the carboxylic acid group such as $-COOR^5$ where $R^5$ is lower alkyl or benzyl. W is preferably $-COOC_2H_5$.

(Step e) Preferably, the conversion of (16) to (17) is by hydrolysis which is carried out by conventional methods such as by an alkali or alkaline earth metal hydroxide such as sodium hydroxide. The reaction is preferably carried out in an inert polar organic solvent such as industrial methylated spirit at ambient temperature.

The racemic ester (16) is prepared by alkylation of the protected piperidine (15) by the corresponding pyrazine (20):

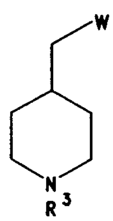

(15)

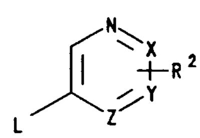

(20)

wherein $R^2$, X, Y, Z, $R^3$ and W are as defined in formula (16); and L is a labile leaving group such as halo (eg. Cl or Br).

(Step f) The alkylation is undertaken in typical conditions such as in the presence of a base such as potassium, lithium or sodium bis(trimethylsilyl)amide, or lithium diisopropyl amide, in a solvent such as tetrahydrofuran or an ether. The reaction is carried out at reduced temperatures such as in the range of from $^{-}10^{\circ}$ - $^{-}30^{\circ}$ C, preferably around $^{-}20^{\circ}$ - $^{-}15^{\circ}$ C.

Preparation of the protected piperidine ester (15) may be undertaken by a series of conventional steps from the commercially-available starting material 4-piperidinone monohydrochloride monohydrate (12).

For example, the corresponding unsaturated ester (14) can be reduced to form (15):

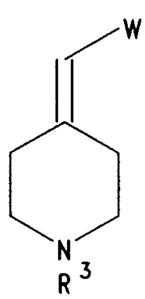

(14)

wherein $R^3$ and W are as defined in formula (15).

(Step g) The reduction may be undertaken by conventional methods such as on 10% palladium/carbon using hydrogen gas or by transfer hydrogenation methods eg. using ammonium formate. The reaction may be carried out in an inert organic solvent such as industrial methylated spirit, preferably at slightly reduced temperature.

The unsaturated ester (14) can be prepared by a Wittig or modified Wittig reaction using the protected piperidinone (13):

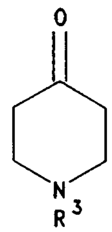

(13)

wherein $R^3$ is as defined in formula (14).

(Step h) This reaction is carried out, for example, when W is $-COOC_2H_5$ by addition of triethylphosphonoacetate in a base such as an alkali metal carbonate (eg. potassium carbonate) in the presence of an inert organic solvent such as dimethyl formamide. Preferably, the addition is under a nitrogen atmosphere and is followed by heating to maintain a temperature of around 70°C.

(Step i) The starting material (4-piperidinone monohydrochloride monohydrate), (12):

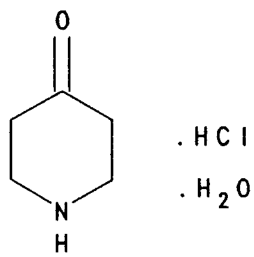

(12)

is protected by conventional techniques for protecting secondary amines such as by addition of $(R^3)_2O$ where $R^3$ is as defined in formula (13), preferably di-tert-butyldicarbonate, but agents such as benzylchloroformate may also be used. The addition is preferably undertaken in aqueous base such as an aqueous inorganic base e.g. a carbonate, hydroxide or hydrogen carbonate e.g. aqueous sodium hydrogen carbonate or an organic base such as triethylamine or DBU.

In any of the above reactions it may be necessary and/or desirable to protect any sensitive groups in the compounds. The protecting groups may be removed at any convenient stage in the synthesis of the desired compound according to conventional techniques.

The present invention further provides novel intermediates of formulae (16), (17), (17A), (18), (19) and (19A) as defined above, and methods for their preparation.

Throughout this specification "lower alkyl" signifies $C_{1-4}$ alkyl groups having straight or branched, saturated chains. Preferably, $R^2$ throughout is hydrogen, halo (preferably chloro or bromo) or lower alkyl (preferably methyl), especially preferred is where $R^2$ is halo such as chloro. The process is particularly suitable where, in formula (I), Z is N, and X and Y are both C. Preferably, $R^2$ is in the 6'-position of the heterocyclic ring. Examples of compounds of formula (I) are the two enantiomers of:

3-(6-chloropyrazin-2-yl)-1-azabicyclo[2.2.2]octane;
3-(6-bromopyrazin-2-yl)-1-azabicyclo[2.2.2]octane; and
3-(6-methylpyrazin-2-yl)-1-azabicyclo[2.2.2]octane; and
salts and prodrugs thereof.

The present invention will now be illustrated by the following example, although other ways of putting it into effect will be clear to the person skilled in the art.

EXAMPLE 1 (R)-(-)-3-(6-Chloropyrazin-2-yl)-1-azabicyclo [2.2.2] octane hydrogen tartrate

[The numbers in the titles refer to those in the Scheme]

A. Preparation of N-tert-butoxycarbonyl piperidin-4-one

To a stirred slurry of sodium bicarbonate (476 g) in water (545 ml) at room temperature was added a solution of 4-piperidinone monohydrochloride monohydrate (12, ex Lancaster Synthesis, 727 g, 4.73 mol) in water (2.47 l) over 20 minutes. To the resultant thin slurry was added di-tert-butyldicarbonate (1.05 kg) in portions over 30 minutes (gas evolution - but no exotherm and little frothing). The mixture was warmed to 35 ° C over 1 hour, aged for 1 hour, then warmed to 50 ° C and held for 2.5 hours.

The mixture was allowed to stir at 50 ° C for a further 30 minutes, cooled to about 30 ° C and ethyl acetate (700 ml) added. The aqueous layer was separated, re-extracted with ethyl acetate (300 ml) and the organic extracts combined. The organic solution was washed with saturated brine solution (300 ml) and then evaporated (Büchi) to yield the product as a colourless solid. The solid was dried in vacuo at room temperature overnight to give the product as a colourless solid, m.p. 74-5 ° C.

B. Preparation of Ethyl-2-[(N-tert-butoxycarbonyl piperidin)-4-ylidene]acetate(14).

Triethylphosphonoacetate (1.42 kg, 6.35 mole) was added to a stirred slurry of milled anhydrous potassium carbonate (2.02 kg, 14.66 mole) in DMF (9.7 l). The piperidinone (13, ex. step 1A) was added in one portion. The resultant mixture was stirred and heated under $N_2$ atmosphere at 70 ° C + 1 ° C for 22 hours in total. Water (30 l) was added dropwise to the cooled reaction mixture (T = 30 ---> 35 ° C). The slurry was aged at 0-5 ° C overnight. The slurry was filtered, the product washed with water (6 l) and dried at room temperature in vacuo overnight. The product was obtained as a colourless solid, m.p. 84.5 - 85.5 ° C.

C. Preparation of Ethyl-2-[(N-tert-butoxycarbonyl piperidin)-4-yl] acetate (15)

To a stirred slurry of unsaturated ester (14, 1.285 kg, 4.77 mole, ex step 1B) in IMS (12.8 l) at 18 ° C under $N_2$ was added a slurry of 10% Pd/C(128 g) in water (400 ml). 10M Ammonium formate solution (930 ml) was added dropwise over 30 minutes. On complete addition, the mixture was stirred for a further 30 minutes. (N.B. slight exotherm noted over about 40 mins, from 18 ---> 24 ° C). After 1 hour GC showed complete reaction. The reaction was filtered through HYFLO which was then washed with IMS (3 1). The filtrate and washings were combined and evaporated to residue. The residue was partitioned between hexane (500 ml) and water (1 l). The organic layer was separated, washed with water (2 x 500 ml) and evaporated to yield a colourless mobile oil which crystallised on standing. The ester (15), was obtained as a colourless solid, m.p. 31 ° C.

D. Preparation of Ethyl-2-[(N-tert-butoxycarbonyl piperidin)-4-yl]-2-(6-chloropyrazin-2-yl) acetate (16)

To a stirred solution of 1M sodium bis(trimethylsilyl) amide in THF (1.66 l, 1.66 mole) at -28 ° C under $N_2$ atmosphere was added a solution of 2,6-dichloropyrazine (115.5 g, 0.77 mole) and ester (15, ex step 1C, 200 g, 0.74 mole) in THF (400 ml) dropwise over about 20 minutes maintaining reaction temperature at -20 ° ---> -15 ° C. On complete addition the reaction was stirred at -10 ° C for 30 minutes. Hexane (400 ml) was added at -10 ° C followed by the dropwise addition of 2M hydrochloric acid (1 l). The lower aqueous phase was separated and the organic phase washed with 2M hydrochloric acid (500 ml) and saturated brine (2 x 500 ml), dried ($MgSO_4$) and evaporated to give the crude product as a dark oil.

E. Preparation and resolution of 2-[(N-tert-butoxycarbonylpiperidin)-4-yl]-2-(6-chloropyrazin-2-yl)acetic acid (17)

A solution of sodium hydroxide pellets (46 g, 1.15 mole) in water (1.6 l) was added to a solution of crude racemic ester (16, ex step 1D, 320 g, 0.738 mole) in IMS (1.6 l) at room temperature. The dark solution was stirred for 1 hour (N.B. small exotherm ---> 30 ° C). After a further 30 minutes at room temperature, HPLC showed complete reaction. The bulk of the IMS was removed in vacuo, T <40 ° C (Büchi) and the aqueous residue was extracted with ethyl acetate (3 x 500 ml). The aqueous solution was acidified with concentrated hydrochloric acid (100 ml) and extracted with ethyl acetate (2 x 500 ml). The organic extracts were combined , washed with brine (300 ml), dried ($MgSO_4$) and evaporated to give crude racemic acid (17) as a gum which solidified on standing. This crude acid (275 g) was dissolved in ethyl acetate (2.0 l) at room temperature and a solution of (L)-(-)-α- methylbenzylamine (56 g, 0.46 mole) in ethyl

acetate (200 ml) added dropwise over about 20 minutes. The resulting slurry was aged at 25° C for 30 minutes, at 50° C for 1 hour and then cooled to room temperature, held for 1 hour and then filtered. The acid salt was washed with ethyl acetate (300 ml) and dried in vacuo at room temperature overnight to give partially resolved (+)-acid salt (17) as a white, crystalline solid, [α]D,20 = +7.8 (c = 0.25, $CH_2Cl_2$). The above salt (180 g) was swished in ethyl acetate (1.8 l) at gentle reflux for 1 hour, cooled to room temperature, aged for 1 hour and filtered. The cake was washed with ethyl acetate (300 ml) and dried in vacuo overnight, [α]D,20 = +12.5°. This swish was repeated to give [α]D,20 = +12.8° (c = 0.25, $CH_2Cl_2$). [Subsequent investigation has shown that an optical rotation of 13.8° is achievable. Hence +12.8° is equivalent to 95.7% major isomer.]

Racemisation of Acid (17) - General Procedure

Trimethylsilylchloride (1.3 equivs.) was added dropwise to a stirred solution of enantiomerically enriched acid (17, 1 equiv.) and triethylamine (3 equivs.) in ethyl acetate (10 mlg-1 relative to acid (17)) at room temperature maintaining temperature at 20-30° C under $N_2$ atmosphere. The resulting slurry was stirred at room temperature for a further 15 minutes and then heated under gentle reflux for 7.5 hours. The reaction mixture was cooled to room temperature and washed with 2M aqueous hydrochloric acid (about 4 mlg-1) relative to acid (17) and (1 mlg-1) then saturated brine (2 x 2 mlg-1), dried ($MgSO_4$) and evaporated to give racemic acid (17) in quantitative recovery.

F. Preparation of (S)-(+)-2-[(N-tert-Butoxycarbonyl piperidin)-4-yl]-2-(6-chloropyrazin-2-yl) ethanol (18)

Resolved (+)-acid salt (17, ex step 1E, 30 g, 63 mmole) was partitioned between ethyl acetate (100 ml) and 1M hydrochloric acid (100 ml). The lower aqueous layer was separated and extracted with ethyl acetate (25 ml). The organic extracts were combined, washed with saturated brine solution (2 x 50 ml), and evaporated to give resolved acid (17, 22.3 g) as a crystalline solid in 100% recovery. The acid (22.3 g) was dissolved in anhydrous THF (67 ml) and cooled to -20° C, under $N_2$ atmosphere with stirring. 1M borane in THF (Aldrich, 189 ml, 3 equivs.) was added dropwise maintaining temperature at -20° C ---> -15° C. The resulting solution was stirred at 0° C for 1 hour and then allowed to warm to room temperature. After a further 2.5 hours, the reaction was complete and was cooled to 0° C. Water (100 ml) was added (carefully) and the mixture stirred vigorously at room temperature until hydrolysis was complete (LC). The THF was evaporated (Büchi) and the aqueous residue extracted with ethyl acetate (3 x 80 ml). The organic extracts were combined, washed with saturated brine solution (80 ml) and evaporated to give an oil. The oil was preabsorbed on silica (50 g), placed on top of clean silica (50 g) in a filter funnel and eluted with 1:1, EtOAc:hexane (6 x 200 ml). Evaporation of the product containing fractions gave alcohol (18) as a viscous oil. [α]D,20 = +50.8° (c = 1.2 in MeOH).

G. Preparation of (S)-(+)-2-[(N-tert-Butoxycarbonyl piperidin)-4-yl]-2-(6-chloropyrazin-2-yl) ethanol methanesulphonate ester (19)

Triethylamine (16.2 ml, 116 mmole) was added to a stirred solution of the alcohol (18, 13.1 g, 38.4 mmole, ex step 1F in ethyl acetate (130 ml) at -20° C under $N_2$ atmosphere. Methanesulphonyl chloride (4.2 ml) was then added dropwise maintaining reaction temperature at -15° C ---> -20° C. The slurry was stirred at -15° C for 30 minutes when 1M hydrochloric acid (100 ml) was added maintaining temperature <0° C. The lower aqueous layer was separated and the organic layer washed with 1M hydrochloric acid (50 ml), 50% aqueous saturated brine solution (2 x 50 ml), dried ($MgSO_4$) and evaporated to give mesylate (19) as a pale yellow foam. [α]D,20 = +22.6° (c = 1, MeOH).

H. Preparation of (R)-(-)-3-(6-chloropyrazin-2-yl)-1-azabicyclo[2.2.2]octane hydrogen tartrate

The crude mesylate (19, 15.9 g, 37.9 mmole, ex step 1G) was dissolved in ethyl acetate (160 ml). Dry hydrogen gas was bubbled through the solution with mechanical stirring at 15-20° C. The deprotection was followed by TLC (Ether/Silica), and was complete after about 2 hours. Water (60 ml) was added to the resulting thick slurry followed by the careful addition of 1:1 wt/wt potassium carbonate/water (80 ml). On complete addition (pH>9) the two phase mixture was heated to 60° C with stirring. The cyclisation was followed by HPLC.
After 4 hours at 60° C, the reaction mixture was cooled to 25° C and the lower aqueous layer was extracted with ethyl acetate (2 x 50 ml) and the organic extracts combined, washed with brine (50 ml) and dried

($MgSO_4$). Evaporation of the solvent gave crude free base as a pale yellow oil. The oil (7.7 g) was dissolved in 1:1 IPA/ethyl acetate (50ml) and added to a warm (50 °C) solution of L-tartaric acid in 1:1 IPA/ethyl acetate (100 ml). The resultant slurry was allowed to cool to room temperature with stirring overnight. The slurry was aged at 0-5 °C for 1 hour, filtered, washed with 1:2, IPA/ethyl acetate (30 ml) and dried in vacuo at room temperature to give tartrate salt as a white, crystalline solid. The salt (9.4 g) was dissolved in methanol (282 ml) at gentle reflux. The solution was cooled slightly and filtered hot. The filter paper was rinsed with methanol (30 ml). The solution was concentrated in vacuo at about 35 °C to a volume of about 100 ml. The resulting slurry was aged at room temperature for 30 minutes, then at 0-5 °C for 1 hour, filtered and washed with cold methanol (20 ml). The semi-purified salt was dried in vacuo at room temperature. The salt was upgraded by swishing in prefiltered refluxing methanol (10 $mlg^{-1}$) for 1 hour. The slurry was cooled to room temperature, held for 1 hour, filtered, washed with methanol and dried in vacuo to give pure tartrate salt, m.p. 175-6 °C, [α]20,405 = $^{+}$62.6°; [α]20,589 = $^{+}$24.5° (c=1, $H_2O$).

| HPLC Profile | | : r.r.t. | Area % |
|---|---|---|---|
| | @ 279 nm | 0.61 | <0.1 |
| | | 1.00 | 99.8 |
| | | 1.44 | <0.2 |
| | @ 209 nm | 0.62 | 0.1 |
| | | 1.00 | 99.6 |
| | | 1.44 | <0.4 |

EXAMPLE 2: Preparation of (-) and (+)-5-[2-(6-bromopyrazin)yl]-1-azabicyclo[2.2.2]octane hydrogen oxalate

(a) A solution of ethyl-2-[(N-tert-butoxycarbonylpiperidin)-4-yl] acetate (53.56g), and 2,6 dibromopyrazine (42.3g, 105mol%) in THF (105ml) was added dropwise over 45 mins to a 1M solution of sodium bis-(trimethylsilyl)amide in THF (490ml, 250mol%) at - 20 °C. The mixture was allowed to warm up to -10 °C on complete addition.

After 2 hours, a further 10mol% $NaN(TMS)_2$ was added (45ml). After 3 hours, the mixture was left stirring at -10 °C for another 30 minutes.

Hexane (200ml) was added followed by 2M HCl (250ml) keeping the temperature below 0 °C. The aqueous layer was removed and the organic layer was washed with brine (3 x 250ml). The solvent was evaporated leaving a dark red gum.

(b) To a solution of the ester prepared in (a) (91.6g) in industrial methylated spirit (800ml) at room temperature was added a solution of sodium hydroxide (12.9g, 150mol%) in water (320ml). The reaction was shown by TLC ($SiO_2/Et_2O$) to be complete after 2 hours. The solvent was removed on a rotary evaporator and water (300ml) was added. The aqueous reaction mixture was extracted with ethyl acetate (3 x 250ml) and neutralised (≈pH2) with 4N sulphuric acid (85ml). The acidic reaction liquors were extracted with EtOAc (250ml + 100ml). The combined organics were washed with brine (2 x 250ml), dried ($MgSO_4$) and treated with charcoal. The solvent was evaporated leaving a sticky brown gum/solid.

(c) At this point, resolution of the isomers was effected in a manner analogous to that in Example 1. The desired isomer was then taken through in a manner analogous to the following steps which refer to the racemate:

(d) A solution of the acid prepared by step (c) (1.10g) in THF (30ml) was cooled to -20 °C. 1M borane/THF solution (75ml, 300mol%) was added over 10 minutes maintaining a temperature of -20 °C. The reaction mixture was allowed to warm to 0 °C and stirred for 1 hour before warming to room temperature. TLC ($SiO_2/Et_2O$) indicated complete reaction after 3 hours. Water (50ml) was added slowly to the cooled reaction mixture at 0 °C. The mixture was stirred at room temperature for 90 minutes. TLC indicated that hydrolysis of the intermediate borate ester was incomplete. Stirring was continued overnight.

The THF was evaporated from the reaction mixture. Water (10ml) was added and the reaction mixture was extracted with ethyl acetate (2 x 40ml). The combined organics were washed with brine

(40ml) and the solvent was evaporated leaving a brown/yellow oil. The crude product was preabsorbed onto silica (24g) and placed on top of clean silica (24g) in a sinter funnel. The silica was eluted under vacuum with 1:1 EtOAc/hexane and the product containing fractions were combined and the solvent evaporated, leaving a darkish yellow oil.

(e) To a solution of the alcohol prepared in Example 2(d) (2.7g) in ethyl acetate (27ml) at -20 °C under $N_2$ was added triethylamine (2.9ml, 21mmol). To the resultant solution at -20 °C was added methanesulphonyl chloride (0.76ml, 9.8mmol) dropwise over 20 minutes. After 30 minutes at -20 °C TLC (diethyl ether/silica) showed complete reaction. 1N sulphuric acid (30ml) was added and the reaction mixture allowed to warm to room temperature. The organic layer was separated and washed with 1N sulphuric acid (20ml), brine (3 x 20ml) and dried ($MgSO_4$). The organics were evaporated to leave the mesylate as an orange oil which slowly crystallised on standing.

(f) To a solution of the mesylate prepared in Example 1(e) (1.5g, 3.23mmol) in ethyl acetate (2ml) at 10 °C was added trifluoroacetic acid (7ml) dropwise over 10 minutes. The mixture was allowed to warm to room temperature and stirred at room temperature for 1 hour. TLC showed complete deprotection (silica/diethyl ether). The solution was cooled to 10 °C and water (10ml) added. Sodium carbonate/water (1:2 wt/wt) was added slowly (effervescence) to pH 9.5. The solution was heated at 60 °C for 4 hours. TLC showed major product component with a trace of base-line (alumina/ethyl acetate/methanol, 10:1). The biphasic mixture was cooled to room temperature and the organic phase separated. The aqueous phase was re-extracted with ethyl acetate (20ml). The combined organics were dried ($MgSO_4$) and evaporated to give an orange oil which darkened quickly on standing. The oil (0.87g) was dissolved in ethyl acetate (10ml) and added to a solution of p-toluenesulphonic acid (1 equiv) in ethyl acetate (15ml). The resultant slurry was stirred at room temperature (sticky solid obtained). IPA (10ml) was added and the mixture stirred for 1 hour and filtered to give an off-white solid.

| (-) Enantiomer:- | | | | |
|---|---|---|---|---|
| calculated: | C, 41.70; | H, 4.25; | N, 10.42; | Br 19.82 |
| found: | C,41.77; | H, 4.28; | N, 10.47; | Br 20.01. |

m.pt. 162-167 °C(dec.)
$[\alpha]_D$-14° (c1.3 in $H_2O$)

| ( + ) enantiomer:- | | | | |
|---|---|---|---|---|
| calculated: | C, 41.70; | H, 4.25; | N, 10.42; | Br 19.82 |
| found: | c,41.62; | H, 4.26; | N, 10.45; | Br, 20.05. |

m.pt. 173-177 °C (dec.)
$[\alpha]$ +15° (c.1.3 in $H_2O$)

**Example 3: Preparation of ( + )- and (-)-5-[2-(6-methylpyrazinyl]-1-azabicyclo[2.2.2]octane hydrogen oxalate**

By a method analogous to that of Examples 1 and 2 may be prepared the title compounds:

| (-) Enantiomer: | | | |
|---|---|---|---|
| calculated: | C, 57.33; | H, 6.53; | N, 14.33 |
| found: | C, 57.25; | H, 6.53; | N, 14.25. |

m.pt. 189-192 °C
$[\alpha]_D^{22}$ = -4.02°

| (+) enantiomer: | | | |
|---|---|---|---|
| calculated:<br>found: | C, 57.33;<br>C, 57.25; | H, 6.53;<br>H, 6.52; | N, 14.33<br>N, 14.20. |

m.pt. 182-192 °C
$[\alpha]_D^{22}$ = +2.93 °

## Claims

1. A process for preparing a substantially pure enantiomer of a compound of formula (I) or a salt or prodrug thereof:

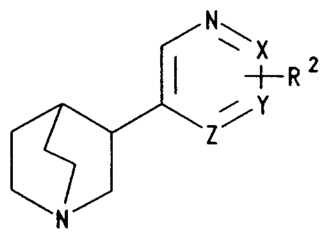

(I)

wherein one of X, Y and Z is an N atom and the remainder are C atoms; and
$R^2$ is hydrogen, halo, -CN, $-COOR^6$, $-CONR^6R^7$, or a saturated or unsaturated, substituted or unsubstituted hydrocarbon group, wherein $R^6$ and $R^7$ are independently selected from hydrogen and $C_{1-2}$ alkyl;
which process comprises cyclisation of an enantiomer of a compound of formula (19A) or salt thereof:

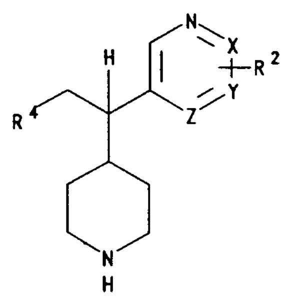

(19A)

wherein
$R^2$, X, Y and Z are as defined in formula (I);
and
$R^4$ is a labile leaving group; and optionally, if desired, converting the compound so prepared to a salt or prodrug thereof.

2. A process according to claim 1 wherein $R^4$ is mesylate ($OS(O)_2CH_3$), lower alkanoate or halo.

3. A process according to claim 1 or claim 2 wherein the cyclisation is carried out in a pH range of from 8 to 10.

4. A process according to claim 3 carried out in a two-phase system comprising a mild inorganic base and a non-aqueous phase selected from any in which the compound of formula (I) so prepared is soluble.

5. A process according to claim 4 wherein the base is selected from an aqueous alkali or alkaline earth metal carbonate, hydrogen carbonate or hydroxide.

**6.** A process according to claim 4 or claim 5 wherein the non-aqueous phase is selected from solvents having a b.p. ≧ 60 ° C.

**7.** A process according to any of claims 1 to 6 which includes the step of resolution of the corresponding racemate of formula (17)

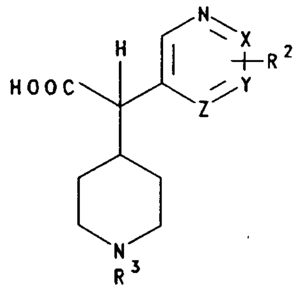

(17)

wherein $R^2$, X, Y, Z and $R^3$ are as defined in formula (17A) to produce the corresponding chiral acid of formula (17A) or a salt thereof:

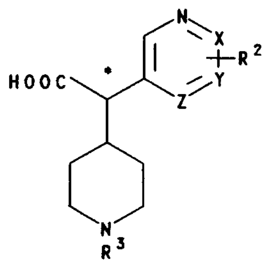

(17A)

wherein $R^2$, X, Y, Z and $R^3$ are as defined in formula (I).

**8.** A process according to claim 7 which uses an optically active isomer of α-methylbenzylamine.

**9.** A process according to any of claims 1 to 8 wherein the compound of formula (I) so prepared is selected from:
(R)-(-)-3-(6-chloropyrazin-2-yl)-1-azabicyclo[2.2.2] octane;
(R)-(-)-3-(6-bromopyrazin-2-yl)-1-azabicyclo[2.2.2] octane; and
(S)-( + )-3-(6-methylpyrazin-2-yl)-1-azabicyclo[2.2.2] octane;
and salts and prodrugs thereof.

**10.** A chiral acid of formula (17A) or a salt thereof:

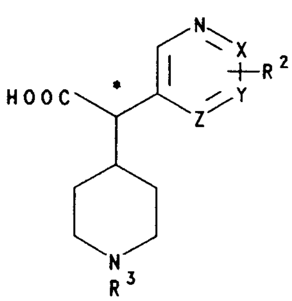

(17A)

wherein $R^2$, X, Y, Z and $R^3$ are as defined in formula (I).

European Patent Office

# EUROPEAN SEARCH REPORT

Application Number

EP 91202005.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP - A2 - 0 327 155 (MERCK SHARP & DOHME LTD) * Abstract; claims 1-6 * -- | 1,9 | C 07 D 453/02 |
| A | EP - A1 - 0 261 763 (BEECHAM GROUP PLC) * Abstract; claims 1,11; page 4, lines 42-45 * ---- | 1 | |
| | | | TECHNICAL FIELDS SEARCHED (Int. Cl.5) |
| | | | C 07 D 453/00 |

The present search report has been drawn up for all claims

| Place of search | Date of completion of the search | Examiner |
|---|---|---|
| VIENNA | 07-10-1991 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document